# EUROPÄISCHE PATENTANMELDUNG

(11) **EP 4 644 701 A1**
(43) Veröffentlichungstag der Anmeldung: **05.11.2025**
(21) Anmeldenummer: 25170526.5
(22) Anmeldetag: 15.04.2025
(51) Int. Cl.: F04D 13/06, F04D 29/42, F04D 29/44, A61M 60/81, A61M 60/104, A61M 60/232, A61M 60/422, A61M 60/822

(54) **PUMPENEINHEIT FÜR EINE ZENTRIFUGALPUMPE SOWIE ZENTRIFUGALPUMPE**

(30) Priorität: 30.04.2024 EP 24173502
(71) Anmelder: Levitronix GmbH, 8048 Zürich (CH)
(72) Erfinder: Steinert, Daniel, 8180 Bülach (CH); Hu, Rennan, 8057 Zürich (CH); Schmid, Alexander, 8915 Hausen am Albis (CH); Barletta, Natale, 8048 Zürich (CH)
(74) Vertreter: IPS Irsch AG

(57) **Zusammenfassung**

Es wird eine Pumpeneinheit für eine Zentrifugalpumpe vorgeschlagen, welche die Pumpeneinheit und einen Stator (100) umfasst, der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche die Pumpeneinheit (1) einsetzbar ist, wobei die Pumpeneinheit (1) ein Pumpengehäuse (2) mit einem Einlass (21) und mit einem Auslass (22) für ein zu förderndes Fluid aufweist, sowie einen in dem Pumpengehäuse (2) angeordneten Rotor (10) mit einer Mehrzahl von Flügeln (103) zum Fördern des Fluids, wobei sich jeder Flügel (103) in axialer Richtung bis zu einer dem Einlass zugewandten Stirnseite (107) des Rotors (10) erstreckt, wobei das Pumpengehäuse (2) einen Pumpenraum (23) begrenzt, wobei der Rotor (10) um die axiale Richtung (A) rotierbar ist, wobei die Pumpeneinheit (1) für eine berührungslos magnetische Lagerung des Rotors (10) und für einen berührungslos magnetischen Antrieb des Rotors (10) durch den Stator (100) ausgestaltet ist, wobei das Pumpengehäuse (2) ein Deckelteil (4) und ein Bodenteil (3) aufweist, wobei das Bodenteil (3) einen zylindrischen Becher (31) zur Aufnahme des Rotors (10) aufweist, welcher Becher (31) in die becherförmige Ausnehmung des Stators (100) einsetzbar ist. Der Einlass (21) weist eine Lippe (28) auf, welche ein axiales Ende des Einlasses (21) bildet, wobei die Lippe (28) in den Pumpenraum (23) hineinragt, und in Strömungsrichtung gesehen von der Stirnseite (107) des Rotors (10) endet, wenn der Rotor (10) im Betriebszustand bezüglich der axialen Richtung (A) zentriert ist. Ferner wird eine Zentrifugalpumpe zum Fördern eines Fluids vorgeschlagen, die eine solche Pumpeneinheit (1) umfasst.

## Beschreibung

Die Erfindung betrifft eine Pumpeneinheit für eine Zentrifugalpumpe gemäss dem Oberbegriff des unabhängigen Patentanspruchs. Die Erfindung betrifft ferner eine Zentrifugalpumpe mit einer solchen Pumpeneinheit.

Es sind Zentrifugalpumpen bekannt, welche eine Pumpeneinheit und einen Stator umfassen, der als Antriebseinheit für den Rotor der Pumpeneinheit ausgestaltet ist, wobei der Rotor der Pumpeneinheit das Flügelrad der Zentrifugalpumpe bildet. Dabei ist der Rotor in der Pumpeneinheit mittels des Stators berührungslos magnetisch lagerbar und berührungslos zur Rotation um eine axiale Richtung antreibbar. Solche Zentrifugalpumpen werden beispielsweise von der Anmelderin unter der Produktbezeichnung Levitronix^{®} BPS Pumpen vertrieben.

Der Stator und der Rotor bilden einen elektromagnetischen Drehantrieb. Bei den Levitronix^{®} BPS Pumpen, beispielsweise, ist der elektromagnetische Drehantrieb nach dem Prinzip des lagerlosen Motors ausgestaltet. Mit dem Begriff lagerloser Motor ist dabei ein elektromagnetischer Drehantrieb gemeint, bei welchem der Rotor vollkommen magnetisch bezüglich des Stators lagerbar ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine durch die axiale Richtung definierte Solldrehachse bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt. In dem Lager- und Antriebsstator lässt sich die Lagerfunktion nicht von der Antriebsfunktion separieren.

Es sind natürlich auch andere Ausgestaltungen von Zentrifugalpumpen bekannt, bei welchen der Rotor berührungslos magnetisch gelagert ist, beispielsweise solche bei denen separate Magnetlager für den Rotor vorgesehen sind, sodass die magnetische Lagerfunktion von der Antriebsfunktion getrennt ist. Beispielsweise sind dazu separate Spulen vorgesehen, mit denen nur die Lagerkräfte für den Rotor realisiert werden, die aber keinen Beitrag zum Antrieb des Rotors leisten. Eine solche Zentrifugalpumpe ist beispielsweise in der WO 2022/004144 offenbart.

Zentrifugalpumpen mit berührungslos magnetisch gelagerten und angetriebenen Rotoren, beispielsweise solche, die nach dem Prinzip des lagerlosen Motors ausgestaltet und betrieben werden, haben sich in einer Vielzahl von Anwendungen bewährt. Aufgrund der Abwesenheit von mechanischen Lagern eignen sich solche Zentrifugalpumpen für Anwendungen, bei denen sehr empfindliche Substanzen gefördert werden, beispielsweise Blutpumpen, oder bei denen sehr hohe Anforderungen an die Reinheit gestellt werden, beispielsweise in der Halbleiterindustrie, der pharmazeutischen Industrie, der biotechnologischen Industrie, oder bei denen abrasive oder aggressive Substanzen gefördert werden, welche mechanische Lager sehr schnell zerstören würden, beispielsweise Pumpen für Slurry, Schwefel-, Phosphorsäure oder andere Chemikalien in der Halbleiterindustrie.

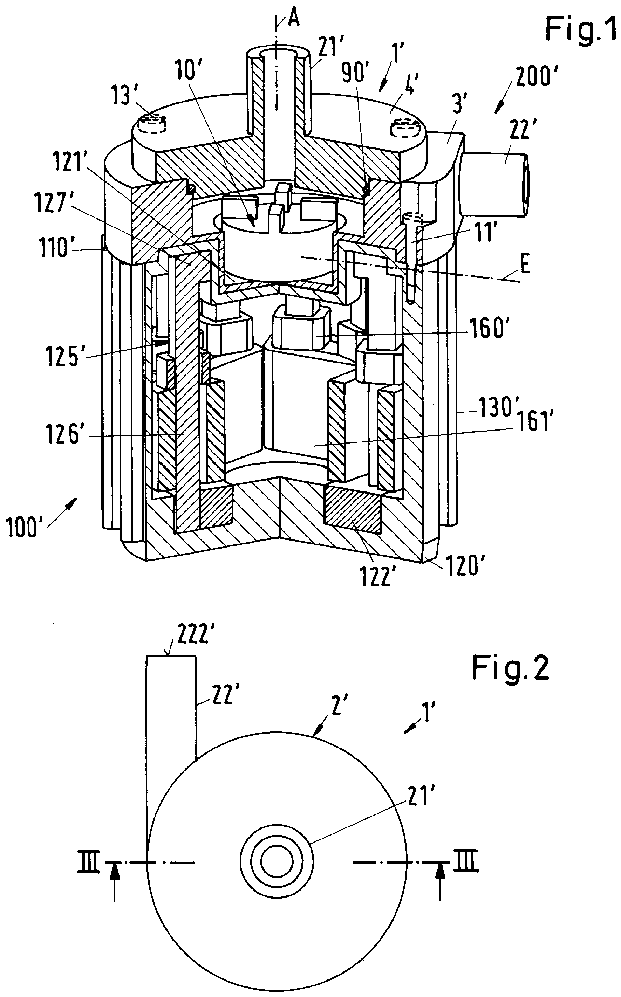

Fig. 1 zeigt eine Darstellung einer aus dem Stand der Technik bekannten Zentrifugalpumpe, die nach dem Prinzip des lagerlosen Motors ausgestaltet ist. Es handelt sich beispielsweise um eine Levitronix^{®} BPS Pumpe. Zum besseren Verständnis ist in Fig. 1 ein Segment herausgeschnitten, damit das Innere der Zentrifugalpumpe sichtbar ist.

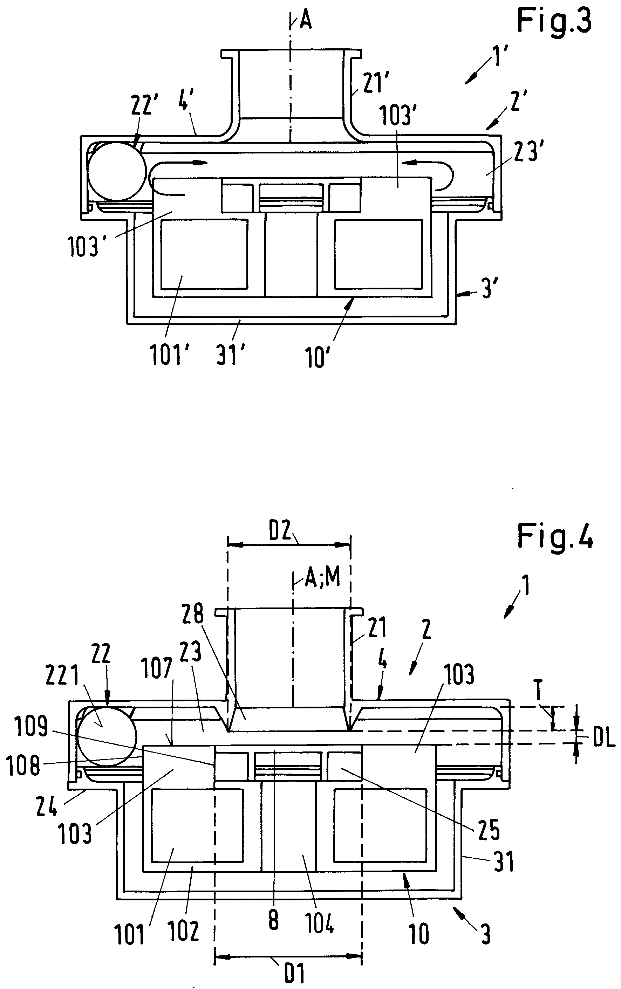

Die Zentrifugalpumpe 200' umfasst einen Stator 100' und eine Pumpeneinheit 1'. Zum besseren Verständnis zeigen Fig. 2 eine Aufsicht auf die Pumpeneinheit 1' aus der axialen Richtung A und Fig. 3 die Pumpeneinheit 1' in einer Schnittdarstellung entlang der Schnittlinie III-III in Fig 2.

Um anzuzeigen, dass es sich bei der Darstellung in Fig. 1, Fig. 2 und Fig. 3 um eine Vorrichtung aus dem Stand der Technik handelt, sind hier die Bezugszeichen jeweils mit einem Hochkomma bzw. mit einem Strich versehen. Die Zentrifugalpumpe ist gesamthaft mit dem Bezugszeichen 200' bezeichnet.

In der Pumpeneinheit 1' ist ein Rotor 10' angeordnet, welcher das Flügelrad bzw. das Laufrad bildet, mit dem das Fluid gefördert wird. Der Stator 100' hat ein Statorgehäuse 130' und erstreckt sich in einer axialen Richtung A von einem ersten axialen Ende 110' bis zu einem zweiten axialen Ende 120', wobei an dem ersten axialen Ende 110' eine becherförmige Ausnehmung 121' vorgesehen ist, in welche die Pumpeneinheit 1' einsetzbar ist. Der Stator 100' bildet mit dem Rotor 10' einen elektromagnetischen Drehantrieb zum Rotieren des Rotors 10' um die axiale Richtung A. Der Stator 100' ist zur berührungslosen magnetischen Lagerung des Rotors 10' nach dem Prinzip des lagerlosen Motors ausgestaltet. Hierzu ist der Stator 100' als Lager- und Antriebsstator ausgestaltet, mit welchem der Rotor 10' berührungslos magnetisch zur Rotation um die axiale Richtung A antreibbar und berührungslos magnetisch bezüglich des Stators 100' lagerbar ist, wobei der Rotor 10' bezüglich der axialen Richtung A passiv magnetisch stabilisiert ist, und in einer zur axialen Richtung A senkrechten radialen Ebene, die in Fig. 1 durch die Linie E angedeutet ist, aktiv magnetisch gelagert ist.

Der elektromagnetische Drehantrieb mit dem Stator 100' und dem Rotor 10' ist als sogenannter Tempelmotor ausgestaltet. Der Stator 100' umfasst eine Mehrzahl von Spulenkernen 125', hier acht Spulenkerne 125', von denen jeder einen Längsschenkel 126' umfasst, welcher sich von einem ersten Ende, in Fig. 1 das darstellungsgemäss untere Ende, in axialer Richtung A bis zu einem zweiten Ende erstreckt, sowie einen Querschenkel 127', welcher an dem zweiten Ende des Längsschenkels 126' und in der radialen Ebene E angeordnet ist. Jeder Querschenkel 127' erstreckt sich von dem zugehörigen Längsschenkel 126' in radialer Richtung auf den Rotor 10' zu und wird durch eine radial innenliegende Stirnfläche begrenzt. Die Spulenkerne 126' sind bezüglich der Umfangsrichtung um die becherförmige Ausnehmung 121' herum angeordnet und damit um den Rotor 10' herum, sodass der Rotor 10' zwischen den radial innenliegenden Stirnflächen der Querschenkel 127' der Spulenkerne 126' angeordnet ist.

Alle ersten Enden der Längsschenkel 126' sind durch einen Rückschluss 122' zur Führung des magnetischen Flusses miteinander verbunden. An jedem Längsschenkel 126' ist mindestens eine konzentrierte Wicklung 160', 161' vorgesehen, welche den jeweiligen Längsschenkel 126' umgibt. Bezüglich Anzahl und Anordnung der konzentrierten Wicklungen 160', 161' sind zahlreiche Varianten bekannt, die hier nicht näher erläutert werden. Es gibt beispielsweise solche Wicklungen 160', die um genau einen Längsschenkel 126' herum gewickelt sind, und solche Wicklungen 161', die um genau zwei Längsschenkel 126' herum angeordnet sind.

Die Mehrzahl der Längsschenkel 126', die sich in axialer Richtung A erstrecken und an die Säulen eines Tempels erinnern, hat dem Tempelmotor seinen Namen gegeben.

Die Pumpeneinheit 1' (Fig. 2, Fig. 3) umfasst ein Pumpengehäuse 2' mit einem Einlass 21' und mit einem Auslass 22' für das zu fördernde Fluid, sowie den in dem Pumpengehäuse 2' angeordneten Rotor 10' zum Fördern des Fluids, der um die axiale Richtung A rotierbar ist. Das Pumpengehäuse 2' begrenzt einen Pumpenraum 23'. Der Rotor 10' umfasst einen magnetisch wirksamen Kern 101', welcher für die Drehmomentbildung sowie für die Erzeugung der magnetischen Lagerkräfte magnetisch mit dem Stator 100' zusammenwirkt. Der magnetisch wirksame Kern 101' ist beispielsweise ein permanentmagnetischer Ring oder eine permanentmagnetische Scheibe.

Im Betriebszustand ist es die Soll-Position des Rotors 10', dass der Rotor 10' bezüglich der axialen Richtung A zentriert ist und in der radialen Ebene E zwischen den Spulenkernen 125', hier den Querschenkeln 127' der Spulenkerne 125' zentriert ist. Bezüglich der axialen Richtung A zentriert bedeutet, dass der magnetisch wirksame Kern 101' des Rotors mit den Querschenkeln 127' der Spulenkerne 125' fluchtet. Die magnetische Mittelebene des Rotors 10' - in der Regel ist dies die geometrische Mittelebene des magnetisch wirksamen Kerns 101' - liegt dann in der radialen Ebene E. Wird der Rotor 10' aus dieser zentrierten Soll-Position in axialer Richtung ausgelenkt oder gegen die axiale Richtung A verkippt, so führt dies zu magnetischen Rückstellkräften, welche den Rotor 10' wieder in seine Soll-Position bewegen.

Es sind auch solche Ausgestaltungen möglich, bei denen der magnetisch wirksame Kern 101' permanentmagnetfrei, also ohne Permanentmagnete ausgestaltet ist. Der Rotor 10' ist dann z.B. als Reluktanzläufer ausgestaltet. Der magnetisch wirksame Kern 101' des Rotors 10' besteht dann beispielsweise aus einem weichmagnetischen Material. Geeignete weichmagnetische Materialien für den magnetisch wirksamen Kern 101' sind beispielsweise ferromagnetische oder ferrimagnetische Materialien, also insbesondere Eisen, Nickel-Eisen, Kobalt-Eisen, Silizium-Eisen, Mu-Metall.

Ferner sind Ausgestaltungen möglich, bei denen der magnetisch wirksame Kern 101' des Rotors 10' sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Typischerweise ist der magnetisch wirksame Kern 101' mit einem Kunststoff vollständig ummantelt. In anderen Ausführungsformen ist der magnetisch wirksame Kern 101' vollständig in einer Ummantelung eingeschlossen, die aus einem keramischen Material oder aus einem metallischen Material, beispielsweise einem rostfreien Stahl oder Titan oder Tantal, besteht.

Der Rotor 10' umfasst ferner eine Mehrzahl von Flügeln 103' zum Fördern des Fluids vom Einlass 21' zum Auslass 22'.

Das Pumpengehäuse 2' umfasst ein Bodenteil 3' und ein Deckelteil 4' zum Verschliessen des Bodenteils 3', wobei zwischen dem Bodenteil 3' und dem Deckelteil 4' ein Dichtungselement 90' (Fig. 1) vorgesehen ist, beispielsweise ein O-Ring oder eine Flachdichtung, um eine Leckage des Fluids in die Umgebung zu vermeiden.

Der Einlass 21' des Pumpengehäuses 2' ist in dem Deckelteil 4' angeordnet und so ausgestaltet, dass das zu fördernde Fluid den Rotor 10' in axialer Richtung A anströmt. Der Auslass 22'erstreckt sich parallel zur radialen Ebene E, also im Wesentlichen senkrecht zum Einlass 21'. Der Einlass 21' ist üblicherweise derart ausgestaltet, dass er einen abgerundeten Übergang in den Pumpenraum 23' darstellt.

Das Bodenteil 3' des Pumpengehäuses 2' weist einen zylindrischen Becher 31' zur Aufnahme des Rotors 10' auf. Der Becher 31' wird in die Ausnehmung 121' im Statorgehäuse 130' eingesetzt, sodass der Rotor 10', genauer gesagt der magnetisch wirksame Kern 101' des Rotors 10' zwischen den Querschenkeln 127' der Spulenkerne 126' angeordnet ist.

Die Pumpeneinheit 1' ist beispielsweise mittels Befestigungselementen 11', z. B. einer Mehrzahl von Schrauben 11', am Statorgehäuse 130' befestigt. Die Schrauben 11' sind an dem Bodenteil 3' angeordnet und fixieren das Bodenteil 3' am ersten axialen Ende 110' des Stators 100'. Das Deckelteil 4' ist üblicherweise über einen Presssitz mit dem Bodenteil 3' verbunden. Zusätzlich wird das Deckelteil 4' mittels mehrerer Befestigungsschrauben 13' am Bodenteil 3' fixiert, welche in axialer Richtung A durch das Deckelteil 4' hindurchgreifen und in das Bodenteil 3' eingreifen.

Für viele Anwendungen, beispielsweise für Anwendungen in der Halbleiterindustrie, wird die Pumpeneinheit 1' - mit Ausnahme des magnetisch wirksamen Kerns 101' - aus einem Kunststoff gefertigt, beispielsweise aus einem Perfluoralkoxy-Polymer (PFA) oder aus Polytetrafluorethylen (PTFE), weil dies Kunststoffe mit einer besonders hohen chemischen Resistenz sind. Diese Kunststoffe sind praktisch inerte Stoffe, die auch von chemisch sehr aggressiven Substanzen, wie sie häufig in der Halbleiterindustrie zum Einsatz kommen, nicht angegriffen werden können. Zudem sind PFA und PTFE sehr reine Kunststoffe, weil sie üblicherweise keine Zusatzstoffe aufweisen und ihre Molekülkomplexe zumindest näherungsweise inert sind. PFA ist häufig bevorzugt, weil es in Spritzgiessverfahren verarbeitet werden kann.

Auch wenn sich diese Art von Pumpeneinheiten 1' und Zentrifugalpumpen 200' in der Praxis sehr gut bewährt hat, so besteht immer noch Verbesserungsbedarf, insbesondere, wenn diese Zentrifugalpumpen 200' für sehr hohe Leistungen ausgestaltet werden. Gerade bei hohen hydraulischen Leistungen tritt ein hoher Leckagestrom auf, der von den Austrittskanten der Flügel 103' in Richtung des Einlasses 21' gerichtet ist. Der Leckagestrom ist in Fig. 3 durch die Pfeile ohne Bezugszeichen angedeutet. Dieser Leckagestrom reduziert den Wirkungsgrad der Zentrifugalpumpe und damit natürlich auch Ihre Energieeffizienz. Zudem versucht der Leckagestrom Abriss-Effekte, insbesondere auch im Bereich des Einlasses, die zu Kavitation und einer weiteren Reduktion des Wirkungsgrads führen können.

Ausgehend von diesem Stand der Technik ist es daher eine Aufgabe der Erfindung, eine Pumpeneinheit mit einem berührungslos magnetisch lagerbaren Rotor für eine Zentrifugalpumpe vorzuschlagen, die insbesondere, aber nicht nur, bei hohen hydraulischen Leistungen einen sehr guten Wirkungsgrad der Zentrifugalpumpe ermöglicht. Zudem ist es eine Aufgabe der Erfindung, eine Zentrifugalpumpe mit einer solchen Pumpeneinheit vorzuschlagen.

Der diese Aufgabe lösende Gegenstand der Erfindung ist durch die Merkmale des unabhängigen Patentanspruchs gekennzeichnet.

Erfindungsgemäss wird also eine Pumpeneinheit für eine Zentrifugalpumpe vorgeschlagen, welche die Pumpeneinheit und einen Stator umfasst, der sich in einer axialen Richtung von einem ersten axialen Ende bis zu einem zweiten axialen Ende erstreckt, wobei an dem ersten axialen Ende eine becherförmige Ausnehmung vorgesehen ist, in welche die Pumpeneinheit einsetzbar ist, wobei die Pumpeneinheit ein Pumpengehäuse mit einem Einlass und mit einem Auslass für ein zu förderndes Fluid aufweist, sowie einen in dem Pumpengehäuse angeordneten Rotor mit einer Mehrzahl von Flügeln zum Fördern des Fluids, wobei sich jeder Flügel in axialer Richtung bis zu einer dem Einlass zugewandten Stirnseite des Rotors erstreckt, wobei das Pumpengehäuse einen Pumpenraum begrenzt, wobei der Rotor um die axiale Richtung rotierbar ist, wobei die Pumpeneinheit für eine berührungslos magnetische Lagerung des Rotors und für einen berührungslos magnetischen Antrieb des Rotors durch den Stator ausgestaltet ist, wobei das Pumpengehäuse ein Deckelteil und ein Bodenteil aufweist, wobei das Bodenteil einen zylindrischen Becher zur Aufnahme des Rotors aufweist, welcher Becher in die becherförmige Ausnehmung des Stators einsetzbar ist. Der Einlass weist eine Lippe auf, welche ein axiales Ende des Einlasses bildet, wobei die Lippe in den Pumpenraum hineinragt, und in Strömungsrichtung gesehen vor der Stirnseite des Rotors endet, wenn der Rotor im Betriebszustand bezüglich der axialen Richtung zentriert ist.

Wenn der Rotor also im Betriebszustand in seiner Soll-Position ist, also bezüglich der axialen Richtung zentriert ist, dann hat die Lippe in axialer Richtung gesehen einen Abstand von der Stirnseite des Rotors, sodass die Lippe nicht in den Rotor hineinreicht. Natürlich ist es aufgrund der magnetischen Lagerung des Rotors möglich, dass sich der Rotor im Betriebszustand gesamthaft in axialer Richtung verschiebt. Durch solche Verschiebungen des Rotors in axialer Richtung ist es im Betriebszustand je nach Ausgestaltung der Lippe möglich, dass die Lippe bezüglich der axialen Richtung in den Rotor eintaucht. Daher bezieht sich die Aussage, dass die Lippe bezüglich der axialen Richtung in Strömungsrichtung gesehen vor der Stirnfläche des Rotors endet, auf einen solchen Betriebszustand, bei welchem der Rotor bezüglich der axialen Richtung zentriert, also in seiner Soll-Position ist.

Die Lippe, welche ein axiales Ende des Einlasses bildet und in den Pumpenraum hineinragt, reduziert deutlich den Leckage Fluss, der von der Austrittskante der Flügel in Richtung des Einlasses 21 fliesst, denn die Lippe reduziert den freien Strömungsquerschnitt für diesen Leckage Fluss, weil sie in den Pumpenraum hineinragt. Alternativ ermöglicht die Lippe eine bezüglich der axialen Richtung höhere Ausgestaltung des Pumpenraums, ohne dass dafür der freie Strömungsquerschnitt für den Leckage Fluss vergrössert wird. Ferner bildet die Lippe eine wohldefinierte Abrisskante für das durch den Einlass einströmende Fluid. Durch diese definierte Abrisskante ist festgelegt, wo die Strömung am Einlass abreisst, und zwar unabhängig von den spezifischen Eigenschaften des Fluids, wie z.B. seiner Viskosität und unabhängig von den spezifischen Strömungsverhältnissen wie z. B. dem Durchfluss. Da der Leckage Fluss von der Austrittskante der Flügel zum Einlass des Pumpengehäuses erheblich reduziert wird, erhöht sich der Wirkungsgrad der Zentrifugalpumpe. Ferner werden durch den Leckage Fluss verursachte Abriss Effekte, die beispielsweise Kavitationen verursachen können, zumindest deutlich reduziert.

Durch die Reduzierung des Leckage Flusses werden auch die in radialer Richtung auf den Rotor wirkenden Störkräfte erheblich reduziert. Hierdurch reduziert sich auch der für die aktive magnetische Radiallagerung des Rotors benötigte elektrische Strom, was den Wirkungsgrad und die Energieeffizienz der Zentrifugalpumpe erhöht.

Ein weiterer Vorteil der als Abrisskante fungierenden Lippe ist es, dass die statischen und dynamischen axial gerichteten Kräfte auf den Rotor reduziert werden, weil die Sogwirkung auf den Deckelteil des Pumpengehäuses verkleinert wird.

Da die Lippe des Einlasses in den Pumpenraum hineinragt, entsteht auch eine ventilartige Wirkung des Einlasses. Diese ist vorteilhaft im Hinblick auf die axiale Stabilisierung des Rotors, sowohl im Hinblick auf Verkippungen des Rotors als auch auf axiale Verschiebungen des Rotors.

Vorzugsweise ist die Lippe ringförmig ausgestaltet und erstreckt sich bezüglich der Umfangsrichtung entlang des gesamten Umfangs des Einlasses. Es sind aber auch Ausgestaltungen möglich, bei denen die Lippe in Umfangsrichtung gesehen mit einem Ausbruch oder mit mehreren Ausbrüchen versehen ist. Beispielsweise kann die Lippe zinnenartig ausgestaltet sein. Die Lippe kann also bezüglich der Umfangsrichtung mit Unterbrüchen ausgestaltet sein.

Gemäss einer bevorzugten Ausgestaltung sind die Flügel des Rotors um einen zentralen Einlassbereich herum angeordnet sind, der sich in axialer Richtung bis an die Stirnseite des Rotors erstreckt, und der einen Durchmesser aufweist. Der zentrale Einlassbereich des Rotors ist frei von Flügeln. Jeder Flügel erstreckt sich von einer radial innenliegend angeordneten Eintrittskante (leading edge) bis zu einer radial aussenliegend angeordneten Austrittskante (trailing edge). Die Eintrittskanten der Flügel liegen auf einer Linie, vorzugsweise einer Kreislinie, die einen von null verschiedenen Abstand von der Mittelachse des Rotors hat. Der Durchmesser des zentralen Einlassbereiches ist durch den Abstand der Eintrittskanten der Flügel von der Mittelachse des Rotors festgelegt. Der Durchmesser des zentralen Einlassbereichs ist gleich dem doppelten Abstand der Eintrittskanten der Flügel von der Mittelachse des Rotors.

Ferner ist es bevorzugt, dass der Rotor mindestens eine Entlastungsöffnung aufweist, die sich von dem zentralen Einlassbereich in axialer Richtung durch den Rotor hindurch erstreckt. Durch diese zentrale Entlastungsöffnung lässt sich der auf den Rotor wirkende Axialschub reduzieren.

Gemäss einer bevorzugten Ausführungsform weist die Lippe des Einlasses einen Aussendurchmesser auf, der grösser ist als der Durchmesser des zentralen Einlassbereichs. Es sind aber auch solche Ausgestaltungen möglich, bei denen der Aussendurchmesser der Lippe kleiner ist als der Innendurchmesser des zentralen Einlassbereichs. Bei diesen Ausgestaltungen ist es dann möglich, dass die Lippe im Betriebszustand durch eine axiale Verschiebung des Rotors in den zentralen Einlassbereich des Rotors eindringt.

Gemäss einer bevorzugten Ausgestaltung weist die Lippe ein im Wesentlichen dreieckiges Profil auf, dessen Spitze der Stirnseite des Rotors zugewandt ist. Hierdurch ist das dem Rotor zugewandte Ende des Einlasses als schmale Kante ausgestaltet, was im Hinblick auf die Funktion als Abrisskante vorteilhaft ist. Es versteht sich, dass bei dem im Wesentlichen dreieckigen Profil die Spitze nicht als scharfe Kante, also nicht im Sinne einer Klinge ausgestaltet ist, sondern als eine Kante mit endlicher Breite.

Ferner ist es eine bevorzugte Massnahme, dass die Lippe sich in Strömungsrichtung gesehen erweitert. Die Querschnittsfläche der Lippe nimmt also in Strömungsrichtung gesehen zu. Beispielsweise kann die Lippe bezüglich der axialen Richtung sich konisch erweiternd ausgestaltet sein.

Auch sind Ausführungsformen möglich, bei welchem die Lippe nach aussen gewölbt ausgestaltet ist.

Eine weitere mögliche Ausgestaltung besteht darin, dass die Lippe als zylindrisches Rohrstück ausgestaltet ist.

Gemäss einer weiteren Ausführungsform ist das Deckelteil des Pumpengehäuses schräg ausgestaltet, sodass das Deckelteil am Einlass einen Winkel mit der axialen Richtung einschliesst, der grösser als 90° ist.

Gemäss einer weiteren bevorzugten Ausgestaltung weist der Auslass eine Eintrittsfläche auf, durch welche das Fluid aus dem Pumpenraum in den Auslass strömen kann, wobei die Eintrittsfläche des Auslasses ein Profil aufweist, welches verschieden von einer Kreisfläche ist. Durch diese Ausgestaltung der Eintrittsfläche des Auslasses kann der in axialer Richtung gemessene Abstand zwischen der Eintrittsfläche und den Flügeln deutlich reduziert werden, ohne dass die Querschnittsfläche der Eintrittsfläche reduziert werden muss. Da das Profil der Eintrittsfläche des Auslasses von einer Kreisfläche verschieden ist, kann die Eintrittsfläche bezüglich der axialen Richtung näher an den Flügeln angeordnet werden als bei einem kreisförmigen Profil der Eintrittsfläche, ohne die Querschnittsfläche der Eintrittsfläche zu reduzieren.

Vergleicht man beispielsweise eine Eintrittsfläche mit einem kreisförmigen Profil, das einen festgelegten Durchmesser aufweist, mit einer Eintrittsfläche mit einem nicht-kreisförmigen Profil, so kann die Eintrittsfläche mit dem nicht-kreisförmigen Profil mit einer grösseren Querschnittsfläche ausgestaltet werden als die Eintrittsfläche mit einem kreisförmigen Profil, ohne dass die maximale Erstreckung der Eintrittsfläche mit dem nicht-kreisförmigen Profil grösser ist als der festgelegte Durchmesser der Eintrittsfläche mit dem kreisförmigen Profil.

Umgekehrt bedeutet dies, dass die Eintrittsfläche mit dem nicht-kreisförmigen Profil im Vergleich zu einer Eintrittsfläche mit kreisförmigem Profil mit der gleichen Querschnittsfläche aber mit einer kleineren Erstreckung insbesondere in axialer Richtung ausgestaltet werden kann. Somit kann die Eintrittsfläche mit dem nicht-kreisförmigen Profil bezüglich der axialen Richtung näher an bzw. mit grösserem Überlapp mit den Flügeln angeordnet werden. Dies wirkt sich besonders vorteilhaft auf die berührungslos magnetische Lagerung des Rotors aus, weil die statischen und dynamischen Axial- und Verkippungskräfte auf den Rotor sowie die in radialer Richtung auf den Rotor wirkenden Störkräfte deutlich reduziert werden.

Gemäss einer bevorzugten Ausgestaltung ist das Profil der Eintrittsfläche im Wesentlichen rechteckförmig ausgestaltet.

Ferner ist es eine insbesondere aus fertigungstechnischen Gründen bevorzugte Ausführungsform, dass das Profil der Eintrittsfläche mit abgerundeten Ecken ausgestaltet ist.

Gemäss einer bevorzugten Ausführungsform weist das Profil der Eintrittsfläche eine Profilhöhe in axialer Richtung auf, sowie eine Profilbreite in einer zur axialen Richtung senkrechten radialen Richtung, wobei die Profilbreite grösser ist als die Profilhöhe.

Durch die Erfindung wird ferner eine Zentrifugalpumpe zum Fördern eines Fluids vorgeschlagen mit einer erfindungsgemäss ausgestalteten Pumpeneinheit, und mit einem Stator, der sich in einer axialen Richtung von einem ersten axialen Ende bis zu einem zweiten axialen Ende erstreckt, wobei an dem ersten axialen Ende eine becherförmige Ausnehmung vorgesehen ist, in welche der zylindrischen Becher der Pumpeneinheit einsetzbar ist, wobei der Stator mit dem Rotor einen elektromagnetischen Drehantrieb zum Rotieren des Rotors um die axiale Richtung bildet, wobei der Stator als Lager- und Antriebsstator ausgestaltet ist, mit welchem der Rotor berührungslos magnetisch antreibbar und berührungslos magnetisch bezüglich des Stators lagerbar ist, wobei der Rotor bezüglich der axialen Richtung passiv magnetisch stabilisiert ist, und in einer zur axialen Richtung senkrechten radialen Ebene aktiv magnetisch gelagert ist.

Besonders bevorzugt ist der elektromagnetische Drehantrieb als Tempelmotor ausgestaltet, wobei der Stator eine Mehrzahl von Spulenkernen aufweist, von denen jeder einen Längsschenkel umfasst, welcher sich von einem ersten Ende in axialer Richtung bis zu einem zweiten Ende erstreckt, sowie einen Querschenkel, welcher an dem zweiten Ende des Längsschenkels und in der radialen Ebene angeordnet ist, und welcher sich von dem Längsschenkel in radialer Richtung erstreckt, wobei die Spulenkerne bezüglich der Umfangsrichtung um den Rotor herum angeordnet sind, sodass der Rotor zwischen den Querschenkeln der Spulenkerne angeordnet ist, und wobei an jedem Längsschenkel mindestens eine konzentrierte Wicklung vorgesehen ist, welche den jeweiligen Längsschenkel umgibt.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der schematischen Zeichnung zeigen (teilweise im Schnitt):
- Fig. 1:: eine perspektivische Darstellung einer Zentrifugalpumpe gemäss Stand der Technik, teilweise im Schnitt,
- Fig. 2:: eine Aufsicht auf die Pumpeneinheit aus der axialen Richtung A,
- Fig. 3: die Pumpeneinheit aus Fig. 2 in einer Schnittdarstellung entlang der Schnittlinie III-III in Fig 2,
- Fig. 4:: ein erstes Ausführungsbeispiel einer erfindungsgemässen Pumpeneinheit in einer Schnittdarstellung entlang der axialen Richtung,
- Fig. 5 - 8, 8A:: verschiedene Varianten für das erste Ausführungsbeispiel, jeweils in einer Darstellung, die derjenigen aus Fig. 4 entspricht,
- Fig. 9:: ein zweites Ausführungsbeispiel einer erfindungsgemässen Pumpeneinheit in einer Schnittdarstellung entlang der axialen Richtung,
- Fig. 10:: eine erste Variante für das Profil der Eintrittsfläche des Auslasses,
- Fig. 11:: eine zweite Variante für das Profil der Eintrittsfläche des Auslasses, und
- Fig. 12:: eine schematische Schnittdarstellung eines Ausführungsbeispiels einer erfindungsgemässen Zentrifugalpumpe.

Wie bereits vorangehend erläutert, zeigt Fig. 1 eine Zentrifugalpumpe 200' mit einem berührungslos magnetisch gelagerten und berührungslos magnetisch angetriebenen Rotor 10', die aus dem Stand der Technik bekannt ist. Fig. 2 und Fig. 3 zeigen in einer Aufsicht bzw. in einer Schnittdarstellung die Pumpeneinheit 1' dieser Zentrifugalpumpe 200'.

Fig. 4 zeigt in einer der Fig. 3 entsprechenden Schnittdarstellung ein erstes Ausführungsbeispiel einer erfindungsgemässen Pumpeneinheit, die gesamthaft mit dem Bezugszeichen 1 bezeichnet ist.

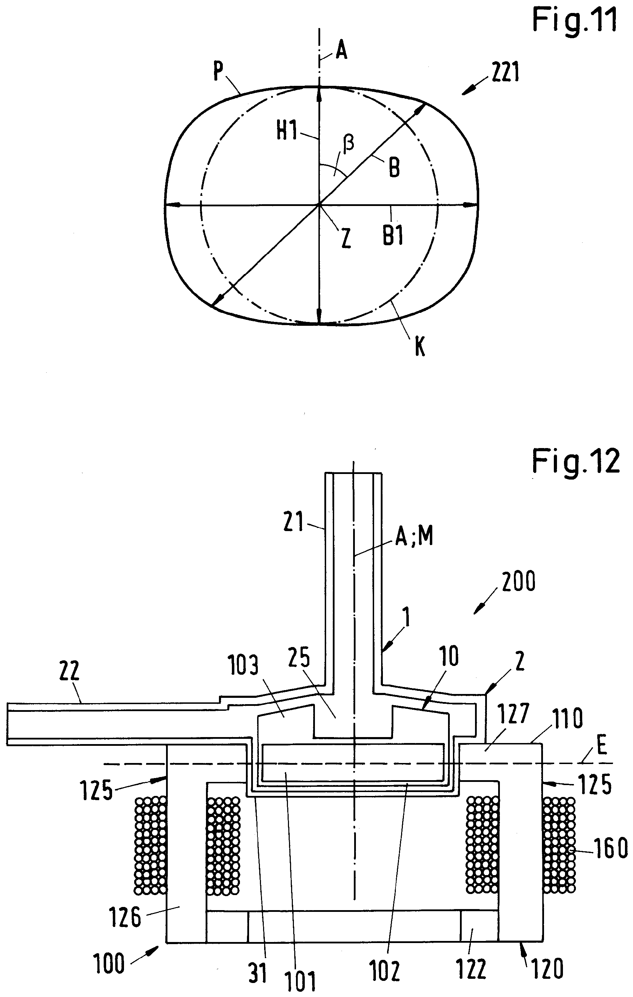

Die Pumpeneinheit 1 ist für eine Zentrifugalpumpe 200 (siehe Fig. 12) zum Fördern eines Fluids ausgestaltet und umfasst ein Pumpengehäuse 2 mit einem Einlass 21 und mit einem Auslass 22 für das Fluid. In dem Pumpengehäuse 2 ist ein Rotor 10 zum Fördern des Fluids angeordnet, welcher das Flügelrad bzw. das Laufrad der Pumpeneinheit 1 und damit der Zentrifugalpumpe 200 bildet. Der Rotor 10 ist um eine Solldrehachse rotierbar, welche eine axiale Richtung A definiert. Diese Solldrehachse ist die Mittelachse M des Rotors 10.

Bezüglich der axialen Richtung A erstreckt sich der Rotor 10 von einer dem Einlass 21 zugewandten Stirnseite 107 bis zu einer dem Einlass 21 abgewandten Rückseite.

Eine zur axialen Richtung A senkrechte Richtung wird als radiale Richtung bezeichnet. Im Folgenden wird der Ausdruck "axial" mit der allgemein üblichen Bedeutung "in axialer Richtung" oder "bezüglich der axialen Richtung" verwendet. Der Begriff "radial" wird mit der allgemein üblichen Bedeutung "in radialer Richtung" oder "bezüglich der axialen Richtung" verwendet.

Die Pumpeneinheit 1 ist für eine berührungslos magnetische Lagerung des Rotors 10 und für einen berührungslos magnetischen Antrieb des Rotors 10 ausgestaltet. Dies kann insbesondere in sinngemäss gleicher Weise realisiert sein, wie dies anhand der Fig. 1 bis Fig. 3 erläutert ist. So kann die erfindungsgemässe Pumpeneinheit 1 bezüglich der magnetischen Lagerung und des magnetischen Antriebs in sinngemäss gleicher Weise ausgestaltet sein wie die Pumpeneinheit 1' in Fig. 3. Dazu umfasst der Rotor 10 der Pumpeneinheit 1 einen magnetisch wirksamen Kern 101, der beispielsweise als permanentmagnetische Ring oder permanentmagnetische Scheibe ausgestaltet ist und von einer Ummantelung 102 umschlossen wird. Die Ummantelung 102 ist vorzugsweise als Kunststoffummantelung ausgestaltet- Die Ummantelung 102 besteht beispielsweise aus PTFE oder PFA. Der magnetisch wirksame Kern 101 ist in der Ummantelung102 eingekapselt, das heisst, die Ummantelung 102 umschliesst den magnetisch wirksamen Kern 101 vollständig und vorzugsweise hermetisch. Dadurch ist der magnetische wirksame Kern 101 vor dem Fluid geschützt. Die Ummantelung 102 kann beispielsweise hergestellt werden, indem der magnetisch wirksame Kern 101 mit einem Kunststoff umspritzt wird.

Der magnetisch wirksamen Kern 101 des Rotors 10 ist diejenige Komponente des Rotors 10, welche für die Drehmomentbildung sowie für die Erzeugung der magnetischen Lagerkräfte magnetisch mit dem Stator 100 zusammenwirkt.

Der Rotor 10 umfasst ferner eine Mehrzahl von Flügeln 103, um das Fluid vom Einlass 21 zum Auslass 22 zu fördern. Die Flügel 103 sind auf der Ummantelung 102 des magnetisch wirksamen Kerns 101 angeordnet. Die Flügel 103 bestehen vorzugsweise aus Kunststoff und können beispielsweise einstückig mit der Ummantelung 102 ausgestaltet sein. Natürlich ist es auch möglich, die individuellen Flügel 103 oder die Gesamtheit der Flügel 103 in einem separaten Fertigungsprozess herzustellen und sie dann mit der Ummantelung 102 des magnetisch wirksamen Kerns 101 zu verbinden, beispielsweise mittels eines Schweissprozesses.

Das von dem Rotor 10 gebildete Laufrad mit den Flügeln 103 ist vorzugsweise als radiales Laufrad ausgestaltet, welches von dem Fluid vom Einlass 21 in axialer Richtung A angeströmt wird, und das Fluid dann in eine radiale Richtung umlenkt.

Das Pumpengehäuse 2 umfasst ein Deckelteil 4 und ein Bodenteil 3, die dichtend miteinander verbunden werden, was in Fig. 4 nicht näher dargestellt ist, weil es für das Verständnis der Erfindung nicht notwendig ist. Das Bodenteil 3 umfasst einen zylindrischen Becher 31 zur Aufnahme des Rotors 10. Der Becher 31 ist vorzugsweise so ausgestaltet und angeordnet, dass er in eine becherförmige Ausnehmung eines Stators 100 einsetzbar ist wie dies in Fig. 12 schematisch dargestellt ist.

Der Stator 100 (Fig. 12) erstreckt sich in der axialen Richtung A von einem ersten axialen Ende 110 bis zu einem zweiten axialen Ende 120 und weist ein Statorgehäuse (in Fig. 12 nicht dargestellt) auf, das im Wesentlichen zylindrisch ausgestaltet ist. Die becherförmige Ausnehmung ist an dem ersten axialen Ende 110 des Stators 100 angeordnet, vorzugsweise zentral in der Stirnfläche, welche das erste axiale Ende 110 des Stators 100 bildet. Die Ausgestaltung des Statorgehäuses, mit der becherförmigen Ausnehmung kann insbesondere in analoger Weise realisiert sein, wie dies anhand der Fig. 1 für das Statorgehäuse 130' und die becherförmige Ausnehmung 121' erläutert wurde. Der Becher 31 ist dann also derart angeordnet und ausgestaltet, dass er in die Ausnehmung 121' (Fig. 1) im ersten axialen Ende 110' des Stators 100' einsetzbar ist, und der magnetisch wirksame Kern 101 zwischen den Querschenkeln 127' der Spulenkerne 125' angeordnet ist.

Wie dies in Fig. 4 zu erkennen ist, befindet sich darstellungsgemäss oberhalb des Bechers 31 ein Pumpenraum 23, welcher durch das Pumpengehäuse 2 begrenzt wird, und in welchem die Flügel 103 des Rotors 10 angeordnet sind. Der Pumpenraum 23 ist zumindest im Wesentlichen zylindrisch ausgestaltet, wobei der Durchmesser des Pumpenraums 23 grösser ist als der Innendurchmesser des Bechers 31. Hierdurch weist das Pumpengehäuse 2 einen flanschartigen Vorsprung 24 auf, welcher den Pumpenraum 23 bezüglich der axialen Richtung A darstellungsgemäss nach unten begrenzt.

Der Einlass 21 ist zentral im Deckelteil 3 des Pumpengehäuses 2 angeordnet, sodass das Fluid den Rotor 10 in axialer Richtung A anströmen kann.

Jeder Flügel 103 erstreckt sich von einer radial innenliegend angeordneten Eintrittskante 109 (leading edge) bis zu einer radial aussenliegend angeordneten Austrittskante 108 (trailing edge). Bei dem hier beschriebenen Ausführungsbeispiel sind die Flügel 103 mit beispielhaftem Charakter so ausgestaltet, dass sie sich geradlinig in radialer Richtung von der Eintrittskante 109 bis zu der Austrittskante 108 erstrecken und über ihre gesamte Erstreckung eine konstante Höhe aufweisen. Mit der Höhe ist dabei die Erstreckung der Flügel 103 in axialer Richtung A gemeint. Diese Ausgestaltung ist - wie gesagt - nur beispielhaft zu verstehen. In anderen Ausführungsbeispielen sind die Flügel 103 z. B. bezüglich der radialen Richtung gekrümmt ausgestaltet und/oder mit einer Höhe in axialer Richtung A, die sich von der Eintrittskante 109 bis zur Austrittskante 108 ändert. In Fig. 12 ist beispielsweise eine Ausgestaltung gezeigt, bei welcher die Flügel 103 an ihrer Eintrittskante 109 eine grössere Höhe aufweisen als an ihrer Austrittskante 108.

Ferner ist optional eine ringförmige Deckplatte 8 vorgesehen, welche auf den dem Einlass 21 zugewandten Oberkanten der Flügel 103 angeordnet ist. Die Deckplatte 8 überdeckt alle Flügel 103. Bezüglich der radialen Richtung erstreckt sich die ringförmige Deckplatte 8 von den Eintrittskanten 109 der Flügel 103 bis zu ihren Austrittskanten 108. Die Deckplatte 8 bildet hier die Stirnseite 107 des Rotors 10. Falls der Rotor 10 ohne die Deckplatte 8 ausgestaltet ist , so bilden die dem Einlass zugewandten Oberkanten der Flügel 103 die Stirnseite des Rotors 10.

Die Flügel 103 des Rotors 10 sind um einen zentralen Einlassbereich 25 herum angeordnet, der frei von Flügeln 103 ist. Die Eintrittskanten 109 der Flügel 103 liegen auf einer Linie, hier einer Kreislinie, die einen von null verschiedenen Abstand von der Mittelachse M des Rotors 10 hat. Der Durchmesser D1 des zentralen Einlassbereiches 25 ist durch den Abstand der Eintrittskanten 109 der Flügel 103 von der Mittelachse M des Rotors 10 festgelegt. Dieser Durchmesser D1 des zentralen Einlassbereichs 25 ist gleich dem doppelten Abstand der Eintrittskanten 109 der Flügel 103 von der Mittelachse des Rotors 10. Bei dem hier beschriebenen Ausführungsbeispiel ist der Durchmesser D1 des zentralen Einlassbereichs 25 gleich gross wie der Innendurchmesser der ringförmigen Deckplatte 8. Bezüglich der axialen Richtung A erstreckt sich der zentrale Einlassbereich 25 entlang der Eintrittskanten 109 der Flügel 103 bis an die Stirnseite 107 des Rotors 10.

Falls die Eintrittskanten 109 der Flügel nicht parallel zur axialen Richtung A verlaufen, sondern beispielsweise gegenüber der axialen Richtung A geneigt sind, so ist der Durchmesser D1 des zentralen Einlassbereichs 25 durch den Abstand der Eintrittskanten 109 an den dem Einlass 21 zugewandten Oberkanten der Flügel 103 festgelegt.

Der Rotor 10 umfasst ferner eine Entlastungsöffnung 104, die kreiszylinderförmig ausgestaltet ist, und sich in axialer Richtung A erstreckt. Die Entlastungsöffnung 104 ist zentral im Rotor 10 angeordnet, sodass die Achse dieser Entlastungsöffnung 104 mit der Mittelachse M des Rotors 10 zusammenfällt. Die Entlastungsöffnung 104 erstreckt sich von dem zentralen Einlassbereich 25 in axialer Richtung A durch den Rotor 10 hindurch bis an die die Rückseite des Rotors 10.

Der Auslass 22 weist eine Eintrittsfläche 221 auf, durch welche das Fluid aus dem Pumpenraum 23 in den Auslass 22 einströmen kann, sowie eine Austrittsfläche (in Fig. 4 nicht zu sehen), durch welche das Fluid den Auslass 22 verlässt. Mit dem Profil der Eintrittsfläche 221 wird wie allgemein üblich, die Querschnittsfläche senkrecht zur Hauptströmungsrichtung des Fluids im Auslass 22 bezeichnet. Das Profil der Eintrittsfläche 221 ist hier, wie in Fig. 4 dargestellt, eine Kreisfläche. Das Profil der Eintrittsfläche 221 ist die senkrechte Projektion der Eintrittsfläche 221 auf eine Ebene, die senkrecht auf der Strömungsrichtung des Fluids im Auslass 22 steht.

Erfindungsgemäss umfasst der Einlass 21 eine Lippe 28, welche ein axiales Ende des Einlasses 21 bildet, wobei die Lippe 28 in den Pumpenraum 23 hineinragt und bezüglich der axialen Richtung A beabstandet von der Stirnseite 107 des Rotors 10 ist, wenn der Rotor 10 im Betriebszustand bezüglich der axialen Richtung A zentriert ist. In Strömungsrichtung des Fluids gesehen endet die Lippe 28 vor der Stirnseite 107 des Rotors 10. Die Lippe 28 ist vorzugsweise ringförmig ausgestaltet.

Aufgrund der magnetischen Lagerung des Rotors 10 ist es im Betriebszustand natürlich möglich, dass sich der Rotor gesamthaft in axialer Richtung verschiebt. Dabei ist es durchaus möglich, dass sich der Rotor 10 in axialer Richtung A derart verschiebt, dass der Abstand der Lippe 28 von der Stirnseite 107 zu null wird, oder dass die Lippe 28 sogar in den zentralen Einlassbereich 25 des Rotors 10 eintaucht. Daher bezieht sich das Merkmal, dass die Lippe 28 beabstandet von der Stirnseite 107 endet, auf den Zustand, wenn der Rotor 10 sich bezüglich der axialen Richtung A in seiner Soll-Position befindet, sodass der Rotor 10 bezüglich der axialen Richtung A bezüglich der Stators 100 zentriert ist. Der Rotor 10 ist in der Zeichnung stets in dieser bezüglich der axialen Richtung A zentrierten Position dargestellt.

Der Abstand der Lippe 28 von der Stirnseite 107 des Rotors 10 in axialer Richtung wird im Folgenden als Lippenabstand DL bezeichnet. Der Lippenabstand DL bezieht sich also auf denjenigen Betriebszustand, wenn der Rotor 10 bezüglich der axialen Richtung A zentriert ist.

Die Lippe 28 dient als Abrisskante für das in den Pumpenraum 23 einströmende Fluid. Durch die Lippe 28 existiert ein wohldefinierter Ort, an welchem der Strömungsabriss des Fluids erfolgt. Hierdurch lassen sich negative Abriss-Effekte, die zu Kavitationen und zur Reduzierung des Wirkungsgrads führen, zumindest deutlich reduzieren. Es versteht sich, dass die Lippe 28 in Umfangsrichtung gesehen nicht durchgängig ausgestaltet sein muss, sondern auch mit Unterbrüchen ausgestaltet sein kann.

Ein weiterer Effekt, der in den Pumpenraum 23 hineinragenden Lippe 28 ist es, dass die Lippe 28 den Leckage Fluss, der von den Austrittskanten 108 der Flügel in Richtung des Einlasses 21 strömt, erheblich reduziert. Die Lippe 28 verkleinert den freien Strömungsquerschnitt zwischen den Austrittskanten 108 der Flügel und dem Einlass 21. Durch diese Reduzierung des Querschnitts, durch welchen der Leckage Fluss strömen kann, wird auch der Leckage Fluss reduziert, sodass weniger Fluid pro Zeit von den Austrittskanten 108 der Flügel 103 zum Einlass rückströmt. Die Reduktion des Leckage Flusses erhöht den Wirkungsgrad der Zentrifugalpumpe 200 und damit auch ihre Energieeffizienz.

Bei dem in Fig. 4 dargestellten ersten Ausführungsbeispiel weist die Lippe 28 ein dreieckiges Profil auf, wobei die Spitze des dreieckigen Profils der Stirnseite 107 des Rotors 10 zugewandt ist. Die Lippe 28 hat in axialer Richtung A eine Tiefe T, welche angibt, wie weit die Lippe 28 vom Deckelteil 4 des Pumpengehäuses 2 in den Pumpenraum 23 hineinragt. Die Ausgestaltung der Lippe 28 mit dem dreieckigen Profil ist vorteilhat für die Funktion der Lippe 28 als Abrisskante. Vorzugsweise ist die Lippe 28 mit dem dreieckigen Profil nicht scharfkantig ausgestaltet, sondern die Spitze des im Wesentlichen dreieckigen Profils ist mit einer endlichen und von null verschiedenen Breite in radialer Richtung ausgestaltet.

Die Lippe 28 hat einen Aussendurchmesser D2, womit der Aussendurchmesser D2 der Lippe 28 an ihrem axialen Ende gemeint ist, welches dem Rotor 10 zugewandt ist. Bei dem in Fig. 4 dargestellten ersten Ausführungsbeispiel ist der Aussendurchmesser D2 der Lippe 28 kleiner als der Durchmesser D1 des zentralen Einlassbereiches 25 des Rotors 10. Daher kann die Lippe 28 in den zentralen Einlassbereich 25 eintauchen, falls der Rotor 10 im Betriebszustand in axialer Richtung A darstellungsgemäss nach oben verschoben wird.

Wie dies in Fig. 4 zu erkennen ist, ist die Lippe 28 vorzugsweise derart ausgestaltet, dass sie sich in Strömungsrichtung erweitert Insbesondere ist die Lippe 28 bei dem ersten Ausführungsbeispiel sich konisch erweiternd ausgestaltet, sodass der Innenraum der Lippe 28 kegelstumpfförmig ausgestaltet ist.

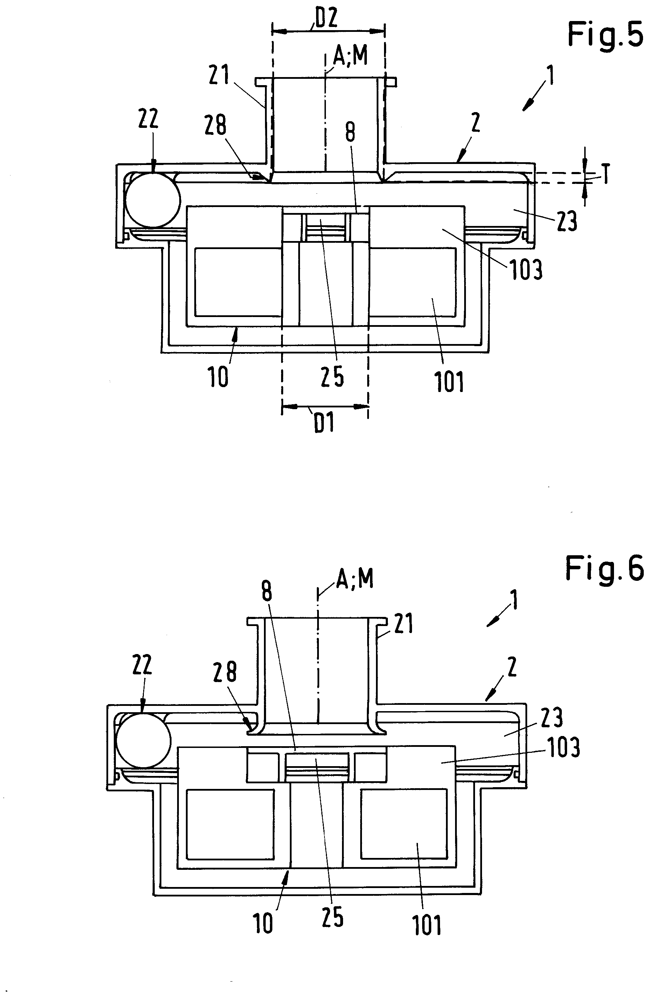

In den Fig. 5 bis Fig. 8 sind verschiedene Varianten für das erste Ausführungsbeispiel der Pumpeneinheit 1 dargestellt, wobei die Darstellung jeweils derjenigen in Fig. 4 entspricht. Fig. 5 bis Fig. 8 sind also jeweils Schnittdarstellungen, wobei der Schnitt entlang der axialen Richtung A erfolgt.

Bei der in Fig. 5 dargestellten Variante hat die Lippe 28 eine geringere Tiefe T, ragt also bezüglich der axialen Richtung A weniger tief in den Pumpenraum 23 hinein. Ferner ist der Aussendurchmesser D2 der Lippe 28 an ihrem axialen Ende, welches dem Rotor 10 zugewandt ist, grösser als der Durchmesser D1 des zentralen Einlassbereiches 25 des Rotors 10. Daher kann die Lippe 28 nicht in den zentralen Einlassbereich 25 eintauchen.

Bei der in Fig. 6 dargestellten Variante ist die Lippe 28 nach aussen gewölbt, sodass sich die Lippe 28 in Strömungsrichtung gesehen wiederum erweitert, jedoch mit einer bezüglich der axialen Richtung A gekrümmt ausgestalteten Wandung.

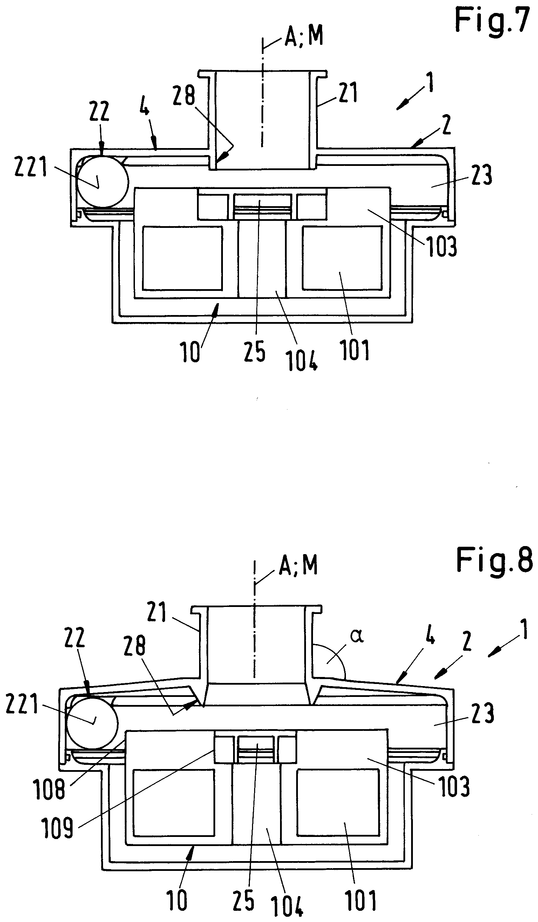

Bei der in Fig. 7 dargestellten Variante ist die Lippe 28 als zylindrisches Rohrstück ausgestaltet, also mit einem in axialer Richtung A konstanten Innendurchmesser. Dabei ist es möglich - wie in Fig. 7 dargestellt, dass die Lippe 28 einstückig mit dem Deckelteil 4 ausgestaltet ist. In anderen Ausgestaltungen kann die Lippe 28 auch als eine separate Komponente ausgestaltet sein, beispielsweise als ein hohlzylindrisches Rohrstück, das in oder an dem Einlass 21 befestigt ist.

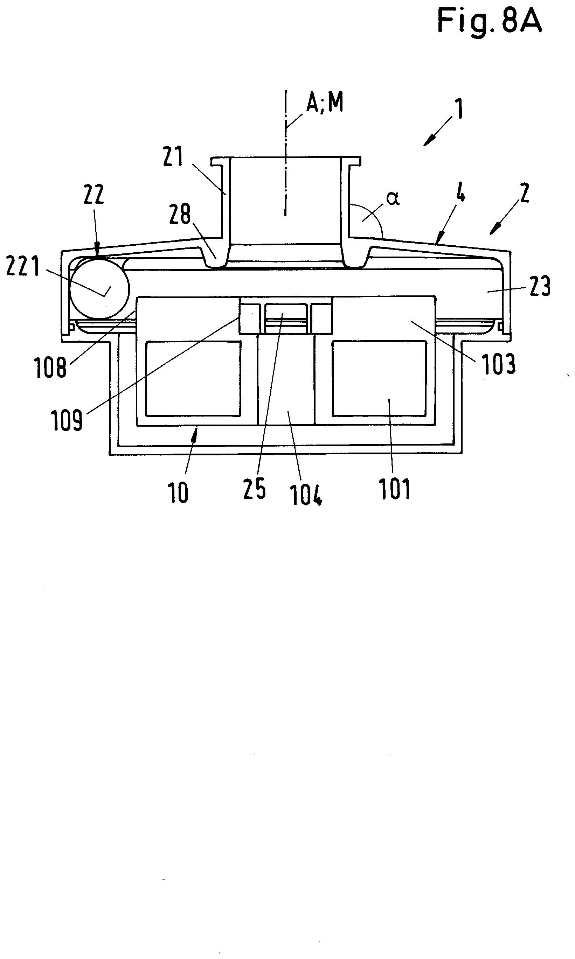

Bei der in Fig. 8 dargestellten Variante ist das Deckelteil 4 des Pumpengehäuses 2 schräg ausgestaltet, sodass das Deckelteil 4 am Einlass 21 mit der axialen Richtung A einen Winkel α einschliesst, der grösser als 90° ist. Der sich an den Einlass 21 anschliessende Bereich des Deckelteils 4 ist also nicht mehr parallel zum Boden des Pumpengehäuses 2, sondern steigt zum Einlass 21 hin an.

Fig. 8A zeigt eine Variante, bei welcher das der Stirnseite 107 des Rotors 10 zugewandte Ende der Lippe 28 stark abgeflacht ausgestaltet ist. Diese Variante ist insbesondere unter fertigungstechnischen Aspekten vorteilhaft.

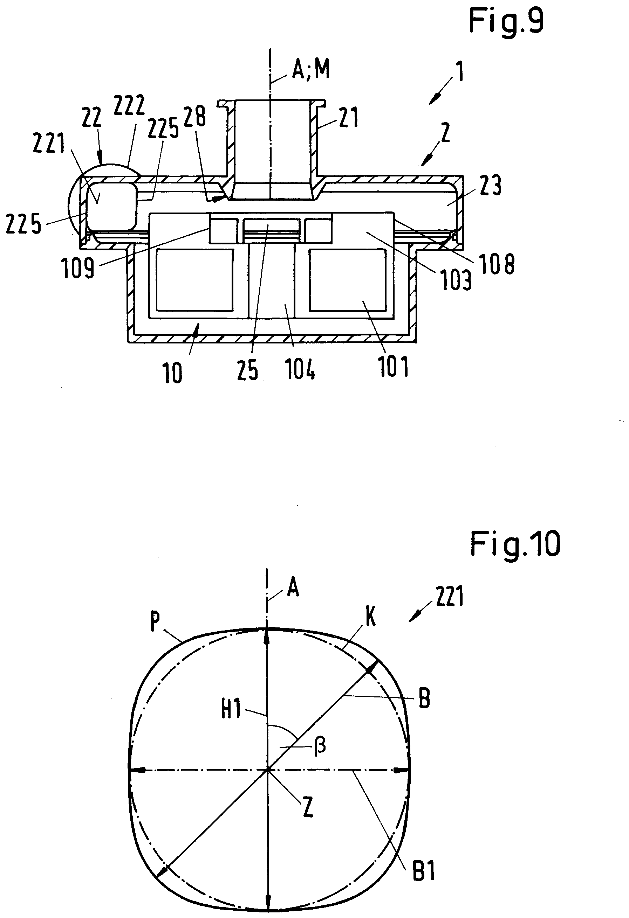

Fig. 9 zeigt ein zweites Ausführungsbeispiel einer erfindungsgemässen Pumpeneinheit 1 in einer Schnittdarstellung, wobei der Schnitt entlang der axialen Richtung erfolgt.

Im Folgenden wird nur auf die Unterschiede zu dem ersten Ausführungsbeispiel eingegangen. Gleiche Teile oder von der Funktion her gleichwertige Teile des zweiten Ausführungsbeispiels sind mit den gleichen Bezugszeichen bezeichnet wie bei dem ersten Ausführungsbeispiel. Insbesondere haben die Bezugszeichen die gleiche Bedeutung wie sie bereits im Zusammenhang mit dem ersten Ausführungsbeispiel erläutert sind. Es versteht sich, dass alle vorangehenden Erläuterungen des ersten Ausführungsbeispiels und seiner Varianten in gleicher Weise oder in sinngemäss gleicher Weise auch für das zweite Ausführungsbeispiel gelten.

Das zweite Ausführungsbeispiel unterscheidet sich von dem ersten Ausführungsbeispiel durch die Ausgestaltung der Eintrittsfläche 221 des Auslasses 22 durch welche das Fluid aus dem Pumpenraum in den Auslass 22 fliesst.

Während bei dem ersten Ausführungsbeispiel (siehe z. B. Fig. 4) das Profil der Eintrittsfläche 221 des Auslasses 22 als Kreisfläche ausgestaltet ist, hat das zweite Ausführungsbeispiel der erfindungsgemässen Pumpeneinheit 1 eine Eintrittsfläche 221 des Auslasses 22, welche verschieden von einer Kreisfläche ist.

Der Auslass 22 hat ferner eine Austrittsfläche 222 (siehe auch Fig. 2), durch welche das Fluid den Auslass 22 verlässt. Wie in Fig. 9 dargestellt, ist das Profil der Austrittsfläche 222 des Auslasses 22 eine Kreisfläche, insbesondere eine Kreisfläche, die eine grössere Querschnittsfläche aufweist als das Profil der Eintrittsfläche. Der Auslass 22 ist somit in Strömungsrichtung gesehen sich erweiternd ausgestaltet, und ändert seine Querschnittsfläche von der nicht-kreisförmigen Eintrittsfläche 221 zu einer kreisförmigen Austrittsfläche.

Bei der in Fig. 9 dargestellten Ausgestaltung ist die Eintrittsfläche 221 des Auslasses so ausgestaltet, dass sie mehrere geradlinige Kanten 225 aufweist. Die Eintrittsfläche 221 ist hier im Wesentlichen rechteckig und im Speziellen im Wesentlichen quadratisch ausgestaltet. Ferner ist es bevorzugt, wenn das Profil der Eintrittsfläche 221 - wie in Fig. 9 dargestellt - mit abgerundeten Ecken ausgestaltet ist. Die Eintrittsfläche 221 mit einem nicht-kreisförmigen Profil also beispielsweise mit mehreren Kanten 225 auszugestalten, hat den Vorteil, dass die Querschnittsfläche der Eintrittsfläche 221 grösser ausgestaltet werden kann als bei einem kreisförmigen Profil, ohne dass dafür die Erstreckung in axialer Richtung A vergrössert werden muss, oder dass die Eintrittsfläche 221 des Auslasses 22 bezüglich der axialen Richtung A näher an die Flügel 103 bzw. mit einem grösseren Überlapp mit den Flügeln 103 angeordnet werden kann.

Die maximale Erstreckung des Profils der Eintrittsfläche 221 in axialer Richtung A wird im Folgenden als Profilhöhe H1 bezeichnet, und die maximale Erstreckung in der dazu senkrechten radialen Richtung als Profilbreite B1.

Ferner sind auch solche Ausgestaltungen des Profils der Eintrittsfläche 221 des Auslasses 22 bevorzugt, bei denen die Profilhöhe H1 kleiner ist als die Profilbreite B1. Beispielsweise kann das Profil der Eintrittsfläche rechteckig ausgestaltet sein, wobei die Profilbreite B1 grösser ist als die Profilhöhe H1. Durch diese Ausgestaltung rutscht der Mittelpunkt des Profils tiefer im Vergleich mit einem kreisförmigen Profil gleicher Fläche. Bei einer solchen Ausgestaltung hat das Profil dann genau vier geradlinige Kanten 225, wobei die Kanten 225, die sich in radialer Richtung erstrecken, länger sind als die Kanten 225, die sich in axialer Richtung A erstrecken. Dabei können die Ecken, welche zwei benachbarte geradlinige Kanten 225 verbinden, abgerundet sein, in sinngemäss gleicher Weise wie das in Fig.9 für das quadratische Profil der Eintrittsfläche 221 dargestellt ist.

Im Folgenden werden anhand der Fig. 10 und Fig. 11 noch zwei Varianten für das Profil der Eintrittsfläche 221 des Auslasses 22 erläutert, bei denen die Eintrittsfläche 221 ein Profil aufweist, das verschieden von einer Kreisfläche ist. Insbesondere sind dabei Ausgestaltungen möglich, bei denen das Profil stärker abgerundet oder auch ganz ohne geradlinige Kanten ausgestaltet ist.

In Fig. 10 ist eine erste Variante für das Profil der Eintrittsfläche 221 dargestellt. Das Profil ist mit dem Bezugszeichen P bezeichnet. Bei dieser ersten Variante ist die Profilhöhe H1 gleich gross wie die Profilbreite B1. Im Vergleich zu der beispielsweise in Fig. 9 dargestellten, im Wesentlichen quadratischen Ausgestaltung des Profils ist das Profil P gemäss Fig. 10 deutlich stärker abgerundet ausgestaltet.

Zum Vergleich ist in Fig. 10 noch eine Kreisfläche K eingezeichnet, deren Durchmesser gleich gross ist wie die Profilhöhe H1 bzw. die Profilbreite B1. Es ist deutlich zu erkennen, dass die Querschnittsfläche des Profils P grösser ist als die Fläche des Kreises K.

Durch das von einer Kreisfläche abweichende Profil P der Eintrittsfläche 221 ist es möglich, das Profil P der Eintrittsfläche 221 des Auslasses 22 mit einer grösseren Querschnittsfläche auszugestalten als bei der kreisförmigen Ausgestaltung des Profils (siehe z.B. Fig. 4), ohne dass dafür die geometrischen Abmessungen des Pumpengehäuses 2 geändert werden müssen. Eine grössere Querschnittsfläche des Profils P der Eintrittsfläche 221 des Auslasses 22 hat den Vorteil, dass der Druckabfall über den Auslass 22, der im Wesentlichen durch diese Querschnittsfläche bestimmt wird, reduziert werden kann, was im Hinblick auf die Effizienz der Pumpeneinheit 1 vorteilhaft ist. Durch den reduzierten Druckabfall über den Auslass 22 erhöht sich der Wirkungsgrad der Zentrifugalpumpe 200.

In Fig. 11 ist eine zweite Variante für das Profil P der Eintrittsfläche 221 dargestellt. Bei der in Fig. 11 dargestellten zweiten Variante ist die Profilbreite B1 grösser als die Profilhöhe H1. Durch diese Ausgestaltung des Profils P verschiebt sich der Mittelpunkt Z des Profils P, oder im allgemeineren Fall eines nicht symmetrischen Profils der Schwerpunkt des Profils P, bezüglich der axialen Richtung A darstellungsgemäss nach unten im Vergleich zu einem kreisförmigen Profil gleicher Querschnittsfläche. Bei dieser Ausgestaltung, bei welcher die Profilbreite B1 grösser ist als die Profilhöhe H1, liegt der Mittelpunkt Z bzw. der Schwerpunkt des Profils P näher an der Ebene, welche die Austrittskanten 108 der Flügel 103 in zwei in axialer Richtung A gleich hohe Abschnitte teilt.

Es sind verschiedene Ausgestaltungen des Profils P möglich, bei denen die Profilbreite B1 grösser ist als die Profilhöhe H1. In Fig. 11 ist beispielsweise eine Ausgestaltung als stark ausgerundetes Rechteck gezeigt. Die Ausrundung des Rechtecks kann dabei so stark sein, dass das Profil P keine im strengen Sinne geradlinigen Kanten mehr umfasst. Weiterhin ist es möglich, das Profil ellipsenförmig auszugestalten. Im Allgemeinen sind bei der zweiten Variante solche Ausgestaltungen bevorzugt, bei denen es mindestens eine zweite Richtung B gibt, in welcher das Profil P eine maximale Erstreckung aufweist, die grösser ist als die Profilhöhe H1, also die maximale Erstreckung des Profils in axialer Richtung A, wobei diese zweite Richtung B mit der axialen Richtung A einen von null verschiedenen Winkel β einschliesst. Bei der Darstellung in Fig. 11 schliesst die zweite Richtung B mit beispielhaftem Charakter den Winkel β = 45° mit der axialen Richtung A ein.

Durch die Erfindung wird ferner die Zentrifugalpumpe 200 zum Fördern eines Fluids mit einer Pumpeneinheit 1 vorgeschlagen, wobei die Pumpeneinheit 1 erfindungsgemäss ausgestaltet ist. Fig. 12 zeigt in einer schematischen Schnittdarstellung ein Ausführungsbeispiel einer erfindungsgemässen Zentrifugalpumpe 200. Die Zentrifugalpumpe 200 umfasst den Stator 100, der sich in der axialen Richtung A von einem ersten axialen Ende 110 bis zu einem zweiten axialen Ende 120 erstreckt, wobei an dem ersten axialen Ende 110 eine becherförmige Ausnehmung (in Fig. 12 nicht dargestellt) vorgesehen ist, in welche der zylindrischen Becher 31 der Pumpeneinheit 1 einsetzbar ist. Der Stator 100 bildet mit dem Rotor 10 einen elektromagnetischen Drehantrieb zum Rotieren des Rotors 10 um die axiale Richtung A, wobei der Stator 100 als Lager- und Antriebsstator ausgestaltet ist, mit welchem der Rotor 10 berührungslos magnetisch antreibbar und berührungslos magnetisch bezüglich des Stators 100 lagerbar ist, wobei der Rotor 10 bezüglich der axialen Richtung A passiv magnetisch stabilisiert ist, und in einer zur axialen Richtung A senkrechten radialen Ebene E aktiv magnetisch gelagert ist.

Der Stator 100 umfasst ein Statorgehäuse, das in Fig. 12 aus Gründen der besseren Übersicht nicht dargestellt ist. Der Stator 100 kann aber beispielsweise in analoger Weise ausgestaltet sein, wie der in Fig. 1 dargestellte Stator 100' mit dem Statorgehäuse 130', wobei in dem Statorgehäuse 130' die Ausnehmung 121' vorgesehen ist, in welche der zylindrische Becher 31 des Bodenteils 3 des Pumpengehäuses 1 eingesetzt wird.

Besonders bevorzugt ist der elektromagnetische Drehantrieb mit dem Rotor 10 und dem Stator 100 als Tempelmotor ausgestaltet, wobei der Stator 100 eine Mehrzahl von Spulenkernen 125 aufweist, von denen jeder einen Längsschenkel 126 umfasst, welcher sich von einem ersten Ende in axialer Richtung A bis zu einem zweiten Ende erstreckt, sowie einen Querschenkel 127, welcher an dem zweiten Ende des Längsschenkels 126 und in der radialen Ebene E angeordnet ist. Der Querschenkel 127 erstreckt sich von dem Längsschenkel 126 in radialer Richtung nach innen auf den Rotor 10 zu.

Alle ersten Enden der Längsschenkel 126 - also die darstellungsgemäss unteren Enden - sind durch einen Rückschluss 122 zum Führen des magnetischen Flusses miteinander verbunden.

Die Spulenkerne 125 sind bezüglich der Umfangsrichtung um den Rotor 10 herum angeordnet sind, sodass der Rotor 10 zwischen den Querschenkeln 127 der Spulenkerne 125 angeordnet ist. An jedem Längsschenkel 126 ist mindestens eine konzentrierte Wicklung 160 vorgesehen, welche den jeweiligen Längsschenkel 126 umgibt.

Mit den konzentrierten Wicklungen 160 werden die für den magnetischen Antrieb und die magnetische Lagerung des Rotors 10 notwendigen elektromagnetischen Felder erzeugt. Mit diesen konzentrierten Wicklungen 160 werden im Betriebszustand also diejenigen elektromagnetischen Felder erzeugt, mit welchen in an sich bekannter Weise ein Drehmoment auf den Rotor 10 bewirkt wird, und mit welchen eine beliebig einstellbare Querkraft in radialer Richtung auf den Rotor 10 ausübbar ist, sodass die radiale Position des Rotors 10, also seine Position in der zur axialen Richtung A senkrechten radialen Ebene E, aktiv steuerbar bzw. regelbar ist. Bezüglich dreier weiterer Freiheitsgrade, nämlich seiner Position in axialer Richtung A und Verkippungen bezüglich der zur Solldrehachse senkrechten radialen Ebene E (zwei Freiheitsgrade), ist der Rotor 10 passiv magnetisch, das heisst nicht ansteuerbar, durch Reluktanzkräfte gelagert bzw. stabilisiert.

## Patentansprüche

1. Pumpeneinheit für eine Zentrifugalpumpe, welche die Pumpeneinheit und einen Stator (100) umfasst, der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche die Pumpeneinheit (1) einsetzbar ist, wobei die Pumpeneinheit (1) ein Pumpengehäuse (2) mit einem Einlass (21) und mit einem Auslass (22) für ein zu förderndes Fluid aufweist, sowie einen in dem Pumpengehäuse (2) angeordneten Rotor (10) mit einer Mehrzahl von Flügeln (103) zum Fördern des Fluids, wobei sich jeder Flügel (103) in axialer Richtung bis zu einer dem Einlass zugewandten Stirnseite (107) des Rotors (10) erstreckt, wobei das Pumpengehäuse (2) einen Pumpenraum (23) begrenzt, wobei der Rotor (10) um die axiale Richtung (A) rotierbar ist, wobei die Pumpeneinheit (1) für eine berührungslos magnetische Lagerung des Rotors (10) und für einen berührungslos magnetischen Antrieb des Rotors (10) durch den Stator (100) ausgestaltet ist, wobei das Pumpengehäuse (2) ein Deckelteil (4) und ein Bodenteil (3) aufweist, wobei das Bodenteil (3) einen zylindrischen Becher (31) zur Aufnahme des Rotors (10) aufweist, welcher Becher (31) in die becherförmige Ausnehmung des Stators (100) einsetzbar ist, **dadurch gekennzeichnet, dass** der Einlass (21) eine Lippe (28) aufweist, welche ein axiales Ende des Einlasses (21) bildet, wobei die Lippe (28) in den Pumpenraum (23) hineinragt, und in Strömungsrichtung gesehen vor der Stirnseite (107) des Rotors (10) endet, wenn der Rotor (10) im Betriebszustand bezüglich der axialen Richtung (A) zentriert ist.

2. Pumpeneinheit nach Anspruch 1, wobei die Flügel (103) des Rotors (10) um einen zentralen Einlassbereich (25) herum angeordnet sind, der sich in axialer Richtung (A) bis an die Stirnseite (107) des Rotors (10) erstreckt, und der einen Durchmesser (D1) aufweist.

3. Pumpeneinheit nach Anspruch 2, wobei der Rotor (10) mindestens eine Entlastungsöffnung (104) aufweist, die sich von dem zentralen Einlassbereich (25) in axialer Richtung (A) durch den Rotor (10) hindurch erstreckt.

4. Pumpeneinheit nach einem der Ansprüche 2-3, wobei die Lippe (28) des Einlasses (21) einen Aussendurchmesser (D2) aufweist, der grösser ist als der Durchmesser (D1) des zentralen Einlassbereichs (25).

5. Pumpeneinheit nach einem der vorangehenden Ansprüche, wobei die Lippe (28) ein im Wesentlichen dreieckiges Profil aufweist, dessen Spitze der Stirnseite (107) des Rotors (10) zugewandt ist.

6. Pumpeneinheit nach einem der vorangehenden Ansprüche, wobei die Lippe (28) sich in Strömungsrichtung gesehen erweitert.

7. Pumpeneinheit nach einem der vorangehenden Ansprüche, bei welchem die Lippe (28) nach aussen gewölbt ausgestaltet ist.

8. Pumpeneinheit nach einem der Ansprüche 1-5, wobei die Lippe (28) als zylindrisches Rohrstück ausgestaltet ist.

9. Pumpeneinheit nach einem der vorangehenden Ansprüche, wobei das Deckelteil (4) des Pumpengehäuses (2) schräg ausgestaltet ist, sodass das Deckelteil (4) am Einlass (21) einen Winkel (α) mit der axialen Richtung (A) einschliesst, der grösser als 90° ist.

10. Pumpeneinheit nach einem der vorangehenden Ansprüche, wobei der Auslass (22) eine Eintrittsfläche (221) aufweist, durch welche das Fluid aus dem Pumpenraum (23) in den Auslass (22) strömen kann, wobei die Eintrittsfläche (221) des Auslasses (22) ein Profil aufweist, welches verschieden von einer Kreisfläche ist.

11. Pumpeneinheit nach Anspruch 10, wobei das Profil (P) der Eintrittsfläche (221) im Wesentlichen rechteckförmig ausgestaltet ist.

12. Pumpeneinheit nach einem der Ansprüche 10-11, wobei das Profil (P) der Eintrittsfläche (221) mit abgerundeten Ecken ausgestaltet ist.

13. Pumpeneinheit nach einem der Ansprüche 10-12, wobei das Profil (P) der Eintrittsfläche (221) eine Profilhöhe (H1) in axialer Richtung (A) aufweist, sowie eine Profilbreite (B1) in einer zur axialen Richtung (A) senkrechten radialen Richtung, wobei die Profilbreite (B1) grösser ist als die Profilhöhe (H1).

14. Zentrifugalpumpe zum Fördern eines Fluids mit einer Pumpeneinheit, die nach einem der vorangehenden Ansprüche ausgestaltet ist, und mit einem Stator (100), der sich in einer axialen Richtung (A) von einem ersten axialen Ende (110) bis zu einem zweiten axialen Ende (120) erstreckt, wobei an dem ersten axialen Ende (110) eine becherförmige Ausnehmung vorgesehen ist, in welche der zylindrischen Becher (31) der Pumpeneinheit (1) einsetzbar ist, wobei der Stator (100) mit dem Rotor (10) einen elektromagnetischen Drehantrieb zum Rotieren des Rotors (10) um die axiale Richtung (A) bildet, wobei der Stator (100) als Lager- und Antriebsstator ausgestaltet ist, mit welchem der Rotor (10) berührungslos magnetisch antreibbar und berührungslos magnetisch bezüglich des Stators (100) lagerbar ist, wobei der Rotor (10) bezüglich der axialen Richtung (A) passiv magnetisch stabilisiert ist, und in einer zur axialen Richtung (A) senkrechten radialen Ebene (E) aktiv magnetisch gelagert ist.

15. Zentrifugalpumpe nach Anspruch 14, bei welchem der elektromagnetische Drehantrieb als Tempelmotor ausgestaltet ist, wobei der Stator (100) eine Mehrzahl von Spulenkernen (125) aufweist, von denen jeder einen Längsschenkel (126) umfasst, welcher sich von einem ersten Ende in axialer Richtung (A) bis zu einem zweiten Ende erstreckt, sowie einen Querschenkel (127), welcher an dem zweiten Ende des Längsschenkels (126) und in der radialen Ebene angeordnet ist, und welcher sich von dem Längsschenkel (126) in radialer Richtung erstreckt, wobei die Spulenkerne (125) bezüglich der Umfangsrichtung um den Rotor (10) herum angeordnet sind, sodass der Rotor (10) zwischen den Querschenkeln (127) der Spulenkerne (125) angeordnet ist, und wobei an jedem Längsschenkel (126) mindestens eine konzentrierte Wicklung (160) vorgesehen ist, welche den jeweiligen Längsschenkel (126) umgibt.
